Europäisches Patentamt

European Patent Office

Office européen des brevets

(11) Veröffentlichungsnummer: **0 670 308 A1**

## (12) EUROPÄISCHE PATENTANMELDUNG

(21) Anmeldenummer: **95102639.2**

(22) Anmeldetag: **24.02.95**

(51) Int. Cl.6: **C07D 201/16**, C07D 201/12

(30) Priorität: **04.03.94 DE 4407222**

(43) Veröffentlichungstag der Anmeldung:
**06.09.95 Patentblatt 95/36**

(84) Benannte Vertragsstaaten:
**AT BE CH DE ES FR GB IT LI NL**

(71) Anmelder: **BASF AKTIENGESELLSCHAFT**

**D-67056 Ludwigshafen (DE)**

(72) Erfinder: **Fuchs, Hugo, Dr.**
**Egellstrasse 28**
**D-67071 Ludwigshafen (DE)**
Erfinder: **Ritz, Josef, Dr.**
**Osloer Weg 8**
**D-67069 Ludwigshafen (DE)**
Erfinder: **Neubauer, Gerald, Dr.**
**Mozartstrasse 24**
**D-69469 Weinheim (DE)**

(54) Verfahren zur Rückgewinnung von Caprolactam aus Oligo- und/oder Polymeren des Caprolactams.

(57) Rückgewinnung von Caprolactam aus Oligo- und/oder Polymeren des Caprolactams durch Spaltung von Oligo- und/oder Polymeren des Caprolactams und anschließende destillative Aufarbeitung des bei der Spaltung erhaltenen Caprolactams, indem man
(a) Oligo- und/oder Polymere des Caprolactam spaltet unter Erhalt eines wäßrigen Reaktionsgemisches, das Caprolactam enthält,
(b) das unter (a) erhaltene Reaktionsgemisch entwässert unter Erhalt eines Rückstandes,
(c) den unter (b) erhaltenen Rückstand im sauren Milieu destilliert und
(d) anschließend das Destillat im alkalischen Milieu unter Erhalt von Caprolactam destilliert, oder
(c') den unter (b) erhaltenen Rückstand im alkalischen Milieu destilliert, und
(d') anschließend das Destillat im sauren Milieu unter Erhalt von Caprolactam destilliert.

EP 0 670 308 A1

Die vorliegende Erfindung betrifft ein Verfahren zur Rückgewinnung von Caprolactam aus Oligo- und/oder Polymeren des Caprolactams durch Spaltung von Oligo- und/oder Polymeren des Caprolactams und anschließende destillative Aufarbeitung des bei der Spaltung erhaltenen Caprolactams.

Bei der Herstellung von Polycaprolactam sowie bei dessen Verarbeitung zu Formteilen wie Fäden, Fasern, Folien, spritzgegossenen oder extrudierten Formteilen fallen Oligomere des Caprolactams und Polycaprolactamabfälle an, die entsorgt werden müssen. Desgleichen müssen die aus Polycaprolactam hergestellten Gebrauchsgüter, wie Folien, Gewebe, Verpackungen, geformte Teile letztendlich entsorgt werden. Hierfür bietet sich die Rückgewinnung von Caprolactam aus Polycaprolactam an.

Polycaprolactam kann in saurem oder in alkalischem Milieu zu Caprolactam gespalten werden. Die in der DE-A 950 726 beschriebene Spaltung mittels Phosphorsäure hat den Nachteil, daß phosphorsäurehaltige Produkte erhalten werden, deren Entsorgung aus Gründen des Umweltschutzes kostenintensiv ist. Die in der DD-A 5310 beschriebenen Spaltung mittels Natriumhydroxid hat den Nachteil, daß das gewonnene Caprolactam im allgemeinen eine schlechte Qualität aufweist und daher aufwendig gereinigt werden muß, um es für die Weiterverarbeitung brauchbar zu machen.

Aufgabe der vorliegenden Erfindung war es daher, ein Verfahren zur Rückgewinnung von Caprolactam durch Spaltung von Oligo- und/oder Polymeren des Caprolactams bereitzustellen, in dem die Nebenprodukte zu Wertprodukten umgesetzt werden.

Demgemäß wurde ein Verfahren zur Rückgewinnung von Caprolactam aus Oligo- und/oder Polymere des Caprolactams durch Spaltung von Oligo- und/oder Polymere des Caprolactams und anschließende destillative Aufarbeitung des bei der Spaltung erhaltenen Caprolactams gefunden, indem man

(a) Oligo- und/oder Polymere des Caprolactams spaltet unter Erhalt eines wäßrigen Reaktionsgemisches, das Caprolactam enthält,

(b) das unter (a) erhaltene Reaktionsgemisch entwässert unter Erhalt eines Rückstandes,

(c) den unter (b) erhaltenen Rückstand im sauren Milieu destilliert und

(d) anschließend das Destillat im alkalischen Milieu unter Erhalt von Caprolactam destilliert, oder

(c') den unter (b) erhaltenen Rückstand im alkalischen Milieu destilliert, und

(d') anschließend das Destillat im sauren Milieu unter Erhalt von Caprolactam destilliert.

Die Spaltung von Oligo- und/oder Polymeren des Caprolactams kann man auf verschiedenen Wegen durchführen. So kann man beispielsweise gemäß der DE-A 950 726 oder der DE-A 12 72 297 Polycaprolactam bei Temperaturen von 200 bis 400°C in Gegenwart von nichtflüchtigen Säuren wie Phosphorsäure in Caprolactam spalten und das entstandene Caprolactam durch überhitzten Wasserdampf aus dem Reaktionsgemisch abdestillieren.

Die Spaltung kann auch gemäß der DD-A 5310 in Gegenwart von Ätzkali bei Temperaturen oberhalb von 250°C bei Drücken von unter 20 mmHg erfolgen.

Eine besondere Ausführungsform besteht darin, daß man je Gewichtsteil Oligo- und/oder Polymere des Caprolactams 5 bis 50, insbesondere 10 bis 20 Gewichtsteile Wasser verwendet und die Umsetzung im allgemeinen bei einer Temperatur von 200 bis 350°C durchführt. Vorteilhaft hält man eine Temperatur von 210 bis 300°C ein. Ferner wird die Spaltung in der Regel unter erhöhtem Druck z.B. von 20 bis 200 bar, insbesondere 20 bis 100 bar, durchgeführt. Es hat sich bewährt, den Druck durch Aufpressen eines inerten Schutzgases, wie Stickstoff zu erhöhen. Vorteilhaft stimmt man Druck und Temperaturbedingungen so aufeinander ab, daß eine flüssige Phase vorliegt.

In der Regel hält man bei der Spaltung Verweilzeiten von 3 bis 6 Stunden ein. Hierbei hat es sich bewährt, daß mindestens 60 Gew. -%, insbesondere 60 bis 90 % des eingesetzten Oligo- und/oder Polymers des Caprolactams in Caprolactam gespalten werden.

Ein wesentliches Merkmal dieser Ausführungsform ist es, daß man die Spaltung unter Mitverwendung von Alkalimetallhydroxiden wie Kaliumhydroxid oder insbesondere Natriumhydroxid bei einem pH-Wert bis 10 durchführt. Besonders bevorzugt wird ein pH-Wert von 6 bis 8 eingehalten.

Nach dem Entspannen erhält man üblicherweise eine wäßrige Lösung oder Suspension, die monomeres Caprolactam, dessen Oligomere sowie gegebenenfalls nichthydrolysiertes Polycaprolactam enthält.

Eine weitere bevorzugte Ausführungsform zur Spaltung von Polycaprolactam besteht darin, daß man Oligo- und/oder Polymere des Caprolactams zunächst im allgemeinen mit 1 bis 20 Gew. -Teilen Wasser, insbesondere 2 bis 10 Gew.-Teilen Wasser je Gew. -Teil Polycaprolactam hydrolytisch spaltet. Hierbei hält man in der Regel eine Temperatur von 200 bis 350°C, insbesondere von 250 bis 300°C, ein. Die Behandlung erfolgt üblicherweise unter erhöhtem Druck, vorteilhaft bei 15 bis 200 bar, wobei der erhöhte Druck zusätzlich durch Aufpressen von Inertgas wie Stickstoff erzeugt wird. Es versteht sich, daß eine flüssige Wasserphase aufrechterhalten wird. Die Behandlung erfolgt im allgemeinen mit einer Verweilzeit von 0,5 bis 10 Stunden, insbesondere 1 bis 5 Stunden. Ferner hat es sich bewährt, zusätzlich Alkalimetallhydroxide, insbesondere Natriumhydroxid in einer Menge von 0,001 bis 0,1 Gew.-Teilen je Gew.-Teil Oligo- und/oder Polymer des Caprolactams mitzuverwenden.

Man erhält im allgemeinen eine wäßrige Reaktionsmischung, die Polycaprolactam, monomeres Caprolactam und dessen Oligomere enthält. Eine typische Zusammensetzung ist beispielsweise 1 bis 70 Gew.-% Caprolactam, 0,1 bis 10 Gew.-% Oligomere des Caprolactams sowie 1 bis 99 Gew.-% suspendiertes Polycaprolactam, bezogen auf eingesetztes Polycaprolactam.

Die so erhaltene Reaktionsmischung wird üblicherweise dann in eine Aluminiumoxid-Wirbelschicht bei einer Temperatur von 270 bis 400°C geleitet, wobei man ein Gemisch aus Wasserdampf und Caprolactam erhält.

Die Reaktionsmischung kann vor Einführen in die Wirbelschicht entspannt werden, zweckmäßig führt man jedoch die Reaktionsmischung über eine Düsenöffnung direkt unter Entspannen in die Wirbelschicht ein. Das Einbringen in die Wirbelschicht kann auch durch Einblasen mittels einer mit einem Inertgas betriebenen Düse erfolgen.

Als Aluminiumoxid eignen sich die verschiedenen Modifikationen wie Tonerde, Böhmit, α- oder γ-Aluminiumoxid. Besonders bewährt hat sich γ-Aluminiumoxid als Katalysator. Der Katalysator wird mit Inertgas wie Kohlendioxid, Argon oder Stickstoff, vorzugsweise Stickstoff, in wirbelnder Bewegung gehalten. Zweckmäßig verwendet man Aluminiumoxid in Korngrößen von 0,05 bis 1,5 mm, insbesondere 0,1 bis 0,4 mm. Die Höhe der Wirbelschicht wird vorteilhaft so gewählt, daß die Verweilzeit des Oligomeren und polymeren Caprolactam in der Katalysatorschicht von 0,1 bis 30, insbesondere von 0,5 bis 10 Sekunden betragen. Vorteilhaft führt man die Behandlung in der Wirbelschicht bei Normaldruck durch. Es kann jedoch auch schwach verminderter oder leicht überhöhter Druck, z.B. von 0,5 bis zu 2 bar angewandt werden.

Vorteilhaft hält man in der Wirbelschicht eine Temperatur von 290 bis 360°C ein. Es ist deshalb auch zweckmäßig, das Inertgas mit einer Temperatur von 290 bis 400°C der Wirbelschicht zuzuführen.

Aus dem aus der Wirbelschicht austretenden Gasgemisch werden die kondensierbaren Anteile durch Kondensieren abgeschieden und dann durch Destillation aufgearbeitet.

Erfindungsgemäß geht man von Oligo- und/oder Polymeren des Caprolactams aus, das entsorgt werden soll, z.B. Abfällen, die bei der Herstellung von Polycaprolactam oder dessen Verarbeitung zu Fäden, Folien, Spritzguß- oder Extrusionsteilen anfallen, ferner geformte Gebrauchsgegenstände wie Folien, Verpackungen, Gewebe, Fäden, extrudierte Teile, die entsorgt werden sollen. Zweckmäßig werden die zu spaltenden Polycaprolactamgegenstände vor der Spaltung zerkleinert, z.B. durch Mahlen, gegebenenfalls vorheriges Verdichten. Vorteilhaft geht man von Polycaprolactam in einer Teilchengröße von 1 bis 100 mm aus.

Das nach der Spaltung erhaltene wäßrige Reaktionsgemisch wird erfindungsgemäß nach an sich bekannten Methoden wie Destillation oder Extraktion, bevorzugt Destillation, entwässert. Bevorzugt führt man das entfernte Wasser, welches üblicherweise wasserdampfflüchtige Verunreinigungen enthält, einer biologischen Abwasserbehandlung zu.

In einer weiteren bevorzugten Ausführungsform behandelt man das nach der Spaltung erhaltene wäßrige Reaktionsgemisch in an sich bekannter Weise vor dem Entwässern mit Kalk, üblichen Oxidationsmitteln wie Kaliumpermanganat und/oder Aktivkohle. Die Gesamtmenge an Kalk, Oxidationsmitteln und Aktivkohle hängt im wesentlichen von der Menge der in dem Reaktionsgemisch vorhandenen Verunreinigungsmengen ab und liegt in der Regel im Bereich von 0 bis 5, bevorzugt von 0,01 bis 1 Gew.-%, bezogen auf das wäßrige Reaktionsgemisch.

Üblicherweise entfernt man nach dieser Behandlung den Feststoffanteil in an sich bekannter Weise, beispielsweise durch Filtration oder Zentrifugieren, vorzugsweise durch Filtration, und entwässert anschließend nach einer der oben genannten Methoden.

Den nach dem Entwässern und ggf. nach Behandlung mit Kalk, Oxidationsmitteln und/oder Aktivkohle erhaltene Rückstand führt man erfindungsgemäß einer Destillation zu. Hierzu versetzt man den Rückstand aus der Reaktionsstufe (b) mit einer Säure oder einem sauren Ionenaustauscher.

Als Säuren kommen üblicherweise Mineralsäuren wie Schwefelsäure, Oleum oder Phosphorsäure und organische Säuren wie p-Toluolsulfonsäure in Frage, bevorzugt Schwefelsäure.

Als saure Ionenaustauscher kann man beispielsweise handelsübliche Ionenaustauscher wie vernetztes Polystyrol mit Sulfonsäure-Gruppen als aktive Gruppen einsetzen.

Die Menge an Säure wählt man im allgemeinen im Bereich von 0,01 bis 5 Gew.-%, vorzugsweise von 0,1 bis 1 Gew.-%, bezogen auf Caprolactam. Die Menge an Ionenaustauscher wählt man in der Regel im Bereich von 1 bis 30, vorzugsweise von 2 bis 10 Gew. -%, bezogen auf Caprolactam.

Die Destillation führt man üblicherweise bei vermindertem Druck, d.h. bei einem Druck von kleiner als 100 kPa, bevorzugt von 300 bis 2000 Pa, aus. Die Temperatur wählt man in der Regel im Bereich von 100 bis 180, vorzugsweise von 120 bis 150°C.

In einer bevorzugten Ausführungsform arbeitet man den Vorlauf, der im wesentlichen leichtsiedende Bestandteile wie Anilin und/oder aliphatische Amide enthält, getrennt auf, bevorzugt zusammen mit dem Verlauf aus der Destillation im alkalischen Milieu (s.u.) . Hierzu führt man vorzugsweise die bei der Destillation im sauren und/oder basischen Milieu erhaltenen Vorläufe einer separaten, üblichen Destillations-Kolonne zu. Besonders vorteilhaft arbeitet man mit einem Rücklaufverhältnis von 1 bis 30, insbesondere von 5 bis 20, wobei man in der Regel 0,1 bis 20, bevorzugt 0,5 bis 10 Gew.-% der eingesetzten Menge als Destillat gewinnt. Vorzugsweise wählt man die Sumpftemperatur im Bereich von 130 bis 160 °C und arbeitet dabei bei einem Druck im Bereich von 400 bis 600 Pa.

Das hierbei erhaltene Destillat wird üblicherweise einer Verbrennungsanlage unter Energiegewinn zugeführt. Den Destillationssumpf, führt man zweckmäßig der Destillationsstufe im sauren Milieu zu.

Den bei der Destillation im sauren Milieu erhaltene Destillationsrückstand führt man erfindungsgemäß einer Spaltschwefelsäureanlage, wenn Schwefelsäure bei der Destillation eingesetzt wird, zur Verbrennung unter Gewinnung von Schwefeldioxid zu. Vorteilhaft kann man den Destillationsrückstand zusammen mit sogenanntem Extraktionswasser ("Raffinat"), wie es aus der Caprolactamherstellung durch Umlagerung von Cyclohexanonoxim nach Beckmann anfällt (s. beispielsweise DE-A 11 94 863), der Verbrennung in einer Spaltschwefelsäureanlage zuführen.

Das Destillat aus der Destillation im sauren Milieu wird üblicherweise bei einer Siedetemperatur im Bereich von 110 bis 130°C abgenommen und einer weiteren Destillation - im alkalischen Milieu - zugeführt. Die Mengen an Destillat liegen in der Regel im Bereich von 1 bis 10 Gew.-%, bezogen auf den eingesetzten entwässerten Rückstand.

Die Durchführung der Destillation im alkalischen Milieu erfolgt beispielsweise wie in der DE-A 31 06 350 beschriebenen Methode, wobei der zu destillierenden Masse ein Alkalimetallhydroxid wie Natrium- oder Kaliumhydroxid, vorzugsweise Natriumhydroxid, in einer Menge im Bereich von 0,01 bis 1, vorzugsweise von 0,01 bis 0,5 Gew.-%, bezogen auf die zu destillierende Masse, zugesetzt wird.

Den Druck während der Destillation wählt man im Bereich von 50 bis 3000 Pa, vorzugsweise von 100 bis 2000 Pa. Das Destillat wird in der Regel bei einer Siedetemperatur im Bereich von 110 bis 130°C entnommen.

In einer bevorzugten Ausführungsform arbeitet man den Vorlauf, der bei der Destillation im alkalischen Milieu anfällt und im wesentlichen leichtsiedende Bestandteile wie Amine, aliphatische Amide und Nitrile enthält, getrennt auf, vorzugsweise zusammen mit dem Vorlauf aus der Destillation im sauren Milieu. Die Vorgehensweise entspricht derjenigen, die bei der Aufarbeitung der Vorläufe aus der Destillation im sauren Milieu beschrieben wurde. Das hierbei erhaltene Destillat wird üblicherweise einer Verbrennungsanlage unter Energiegewinn zugeführt. Den Destillationssumpf, enthaltend üblicherweise im wesentlichen Caprolactam und Methylcaprolactam, führt man zweckmäßig der Destillationsstufe im sauren Milieu zu.

Die Rückstände der Destillation im alkalischen Milieu werden in einer bevorzugten Ausführungsform destillativ aufgearbeitet. Hierzu werden diese in einer üblicherweise mit Einbauten, z.B. Sulzerpackungen, Pallringen, Raschigringen, versehenen Destillationskolonne bei einem Druck bevorzugt im Bereich von 300 bis 1000 Pa und einer Sumpftemperatur im Bereich bis 160 °C, destilliert. Vorteilhaft betreibt man die Destillationskolonne unter leichtem Rückfluß. Man kann die Destillation jedoch auch beispielsweise in einem Dünnschichtverdampfer durchführen. Das Destillat dieser Aufarbeitungsstufe führt man zweckmäßig der Destillation im sauren Milieu, die Destillationsrückstände, die im wesentlichen Alkalimetallverbindungen enthalten, vorzugsweise einer Verbrennungsanlage zu. Bei der Verbrennung erhält man in der Regel Soda als Wertprodukt sowie nutzbare Wärmeenergie.

Das Destillat, das man bei der Destillation im alkalischen Milieu erhält, besteht im allgemeinen aus 98 bis 99,9 Gew.-% Caprolactam.

Falls eine weitere Reinigung des so erhaltenen Caprolactams erforderlich erscheint, kann man dies durch bekannte Methoden wie Kristallisation (s. EP-A-279 439) und/oder durch Vermischen des erfindungsgemäß erhaltenen Caprolactams mit Caprolactam aus der Beckmannschen Umlagerung und gemeinsamer - üblicher - weiterer Reinigung erreichen.

Im übrigen ist es nach den bishierigen Beobachtungen von untergeordneter Bedeutung, ob man das entwässerte Reaktionsgemisch aus der Oligomeren- bzw. Polycaprolactamspaltung zuerst der Destillation im sauren und dann der Destillation im alkalischen Milieu oder in umgekehrter Reihenfolge zuführt.

Das erfindungsgemäße Verfahren zur Spaltung und Aufarbeitung von Polycaprolactam führt sämtliche Rückstände einer Wiederverwertung und/oder einer kontrollierten Entsorgung zu und liefert Caprolactam in einer Reinheit wie es zur Polymerisation von Caprolactam erforderlich ist. Das erfindungsgemäße Verfahren ist daher umweltfreundlich und Rohstoff sparend. Es eignet sich daher zur Entsorgung von Oligo- und/oder Polymeren des Caprolactams enthaltenden Massen wie Teppichen, Ausgüssen, Borsten oder anderen handelsüblichen Formmassen auf der Basis von Polycaprolactam.

Beispiel 1

10.000 g entwässertes Caprolactam aus einer Spaltanlage wurden mit 0,3 Gew.-% Schwefelsäure in einer mit Raschig-Ringen gefüllten Kolonne bei 500 Pa destilliert. Das eingesetzte Caprolactam setzte sich aus 7787 g Caprolactam aus einer Spaltanlage, 1263 g aus dem Sumpf einer Schwersiederabtrennkolonne erhaltenem Caprolactam und 950 g Caprolactam, welches aus einer Leichtsiederabtrennkolonne erhalten wurde, zusammen.

Bei dieser Destillation im sauren Milieu wurden 600 g Destillat als Vorlauf abgezogen. Die angefallene Rückstandsmenge betrug 300 g. Diese wurde unter $SO_2$-Gewinn verbrannt.

Die Hauptmenge der Destillation im sauren Milieu (9100 g) wurde in einer zweiten Kolonne mit Sulzerpackung unter Zusatz von 0,25 Gew.-% Natriumhydroxid destilliert. Die Destillation erfolgte bei 500 Pa und einer Sumpftemperatur von 130°C. Bei dieser Destillation wurden 455 g Destillat als Vorlauf entnommen. Die Vorläufe der Destillation im sauren und im alkalischen Milieu wurden gemeinsam in einer Kolonne bei einem Rücklaufverhältnis von 20 bei einem Druck von 500 Pa destilliert. Hierbei wurde wiederum ein Vorlauf von ca. 10 Gew.-%, bezogen auf die eingesetzte Menge, entnommen und verbrannt. Der Sumpf dieser Vorlaufdestillation (950 g) wurde wieder der Destillation im sauren Milieu zugeführt.

Der Hauptlauf der 2., unter alkalischen Bedingungen durchgeführten Destillation (7280 g) bestand aus als "Reinlactam" bezeichnetes Caprolactam mit folgenden Qualitätskennzahlen:

| | |
|---|---|
| Permanganatabsorptionszahl [1] | 3,6 |
| Extinktion [1 cm Küvette/290 nm] einer 50 gew.-%igen wäßrigen Lösung | 0,028 |
| freie Basen [meq/kg] | 0,01 |
| flüchtige Basen [meq/kg] | 0,29 |
| Oktahydrophenazin [mg/kg] | 0,5 |
| Farbzahl APHA* | < 1 |

[1] s. auch Caprolactam-Broschüre der BASF oder ISO-Standards Caprolactam

*) Die APHA-Farbzahl (s. Römpps Chemielexikon, Bd. 2, 8. Auflage, S. 1243) wurde an einer 10 gew.-%igen Lösung des Caprolactams in N-Methylpyrrolidon in einem Spektrometer (Beckmann, DU, Series 60) bestimmt.

Die bei der unter alkalischen Bedingungen durchgeführten 2. Destillation angefallene Sumpfmenge von 1365 g wurde ihrerseits bei einem Druck von 500 Pa durch Destillation aufgearbeitet. Ein Rückstand von 102 g enthielt das gesamte zugesetzte NaOH und wurde unter Sodagewinnung verbrannt. Das Destillat von 1263 g wurde der Destillation im sauren Milieu zugeführt (s.o.).

Zur photometrischen Bestimmung der Permanganatabsorptionszahl wurden je 1,000 g der zu untersuchenden Mischung in einem 100-mL-Meßkolben gegeben.

Anschließend wurde destilliertes, sauerstofffreies Wasser von pH 6,2 - 6,5 bis zur Eichmarke aufgefüllt und gut durchmischt. Ein zweiter 100-mL-Meßkolben wurde nur mit destilliertem, sauerstofffreiem Wasser von pH 6,2 - 6,5 bis zur Eichmarke gefüllt. Nach dem Temperieren beider Kolben (ca. 30 min) bei 25°C in einem Thermostaten wurden zuerst zum Wasser und unmittelbar danach zur Caprolactam-haltigen Lösung je 2,00 mL einer 0,01 N Kaliumpermanganatlösung zugesetzt. Bei Beginn der Kaliumpermanganatzugabe wurde eine Stoppuhr in Gang gesetzt. Beide Meßkolben wurden sofort verschlossen, gut gemischt und wieder in den Thermostaten gestellt. Nach ca. 9 min wurden zwei Küvetten der Länge l = 5 cm mit den beiden Lösungen gefüllt und in ein Spectralphotometer eingesetzt. 10 Minuten ±10 sec nach Zugabe der Kaliumpermanganatlösung wurde die Extinktion E bei 420 nm der Caprolactamlösung gegen das permanganatbehandelte Wasser gemessen. Die Permanganatabsorptionszahl ergibt sich sodann aus der Differenz der Extinktionswerte beider Lösungen multipliziert mit Faktor 100.

Die Bestimmung des Gehaltes an flüchtigen Basen wurden nach der Kjeldahl-Methode in einer Apparatur nach Parnas durchgeführt. Hierzu wurden pro Messung jeweils 100 mL 4mol/l Natronlauge in den Kjeldahl-Kolben der Apparatur eingefüllt und 20±0,1 g der zu untersuchenden Lösung mit insgesamt 70 mL destilliertem Wasser hinzugefügt. Dann wurden unter Einblasen von Wasserdampf durch die Lösung des Kjeldahl-Kolbens innerhalb von ca. 5 min 50 ml des Kolbeninhalts in eine Vorlage, die 5,00 mL 0,02 mol/l Salzsäure, 30 mL destilliertes Wasser und 5 Tropfen einer Indikatorlösung (hergestellt aus 0,3 g Methylrot und 0,3 g Methylenblau in 400 ml Methanol) destilliert. Der Überschuß an Salzsäure in der Vorlage wurde mit 0,02 mol/l Natronlauge (Verbrauch: A mL) zurücktitriert. Eine Blindwertbestimmung ohne Zugabe einer Caprolactamlösung ergab den Gehalt an flüchtigen Basen der verwendeten Natronlauge (Verbrauch: B mL). Aus (B-A)•0,02•1000/20 = B - A meq/kg ließ sich sodann der Gehalt an flüchtigen Basen errechnen.

Vergleichsbeispiel

Das obengenannte Beispiel wurde wiederholt, mit dem Unterschied, daß bei der ersten Destillation (Destillation im sauren Milieu bei o.g. Beispiel) keine Schwefelsäure zugesetzt wurde.

Der Hauptlauf der 2., unter alkalischen Bedingungen durchgeführten Destillation bestand aus einem Caprolactam mit folgenden Qualitätskennzahlen

| | |
|---|---|
| Permanganatabsorptionszahl | 12 |
| Extinktion [1 cm Küvette/ 290 nm] einer 50 Gew.-%igen wäßrigen Lösung | 0,16 |
| flüchtige Basen [meq/kg] | 0,3 |
| freie Basen meq/kg | 2,8 |
| Oktahydrophenazingehalt [mg/kg] | 1 |
| Farbzahl APHA | 6 |

**Patentansprüche**

1. Verfahren zur Rückgewinnung von Caprolactam aus Oligo- und/oder Polymeren des Caprolactams durch Spaltung von Oligo- und/oder Polymeren des Caprolactams und anschließende destillative Aufarbeitung des bei der Spaltung erhaltenen Caprolactams, dadurch gekennzeichnet, daß man
    (a) Oligo- und/oder Polymere des Caprolactams spaltet unter Erhalt eines wäßrigen Reaktionsgemisches, das Caprolactam enthält,
    (b) das unter (a) erhaltene Reaktionsgemisch entwässert unter Erhalt eines Rückstandes,
    (c) den unter (b) erhaltenen Rückstand im sauren Milieu destilliert und
    (d) anschließend das Destillat im alkalischen Milieu unter Erhalt von Caprolactam destilliert, oder
    (c') den unter (b) erhaltenen Rückstand im alkalischen Milieu destilliert,
    (d') und anschließend das Destillat im sauren Milieu unter Erhalt von Caprolactam destilliert

2. Verfahren gemäß Anspruch 1, dadurch gekennzeichnet, daß man direkt im Anschluß an die Spaltung das wäßrige Reaktionsgemisch mit Kalk, Oxidationsmitteln und/oder Aktivkohle behandelt.

3. Verfahren nach Anspruch 2, dadurch gekennzeichnet, daß man nach der Behandlung mit Kalk, Oxidationsmitteln und/oder Aktivkohle den Feststoffanteil abtrennt und die flüssige Phase entwässert.

4. Verfahren gemäß den Ansprüchen 1 bis 3, dadurch gekennzeichnet, daß man bei der Destillation im sauren und/oder alkalischen Milieu einen Vorlauf entnimmt und diesen destillativ in einen leichtsiedenden Anteil, der einer Verbrennungsanlage zugeführt wird, und einen Sumpfanteil, der in die Destillationsstufe im sauren Milieu zurückgeführt wird, auftrennt.

5. Verfahren gemäß den Ansprüchen 1 bis 4, dadurch gekennzeichnet, daß man den bei der Destillation im sauren Milieu erhaltenen Rückstand einer Spaltschwefelsäureanlage zur Verbrennung zuführt, falls die Destillation in Gegenwart von Schwefelsäure durchgeführt wird.

6. Verfahren gemäß den Ansprüchen 1 bis 5, dadurch gekennzeichnet, daß man den bei der Destillation im alkalischen Milieu erhaltenen Rückstand destillativ auftrennt, wobei man das hierbei erhaltene Destillat in die Destillationsstufe im sauren Milieu zurückführt und den Rückstand einer Verbrennungsanlage zuführt.

7. Verwendung eines der Verfahren gemäß den Ansprüchen 1 bis 6 zur Entsorgung von Oligo- und/oder Polymeren des Caprolactams enthaltenden Massen.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | US-A-3 600 381 (A. YAMAMOTO ET AL) <br> * Ansprüche; Beispiele 1-3 * <br> --- | 1-3,7 | C07D201/16 <br> C07D201/12 |
| Y | DE-C-739 953 (I.G. FARBENINDUSTRIE AG) <br> * Seite 2, Spalte 1, Zeile 9 - Zeile 14; Ansprüche 1,3; Beispiel 2 * <br> --- | 1-3,7 | |
| A | DATABASE WPI <br> Week 8651 <br> Derwent Publications Ltd., London, GB; <br> AN 86-338250 <br> & SU-A-1 231 052 (SYNTH FIBRE RES INS) <br> * Zusammenfassung * <br> --- | 1-3,7 | |
| A | PATENT ABSTRACTS OF JAPAN <br> vol. 3 no. 128 (C-62) ,24.Oktober 1979 <br> & JP-A-54 109992 (TOYO BOSEKI KK) <br> * Zusammenfassung * <br> --- | 1-3,7 | |
| A | PATENT ABSTRACTS OF JAPAN <br> vol. 7 no. 7 (C-144) ,12.Januar 1983 <br> & JP-A-57 165364 (UNITIKA KK) <br> * Zusammenfassung * <br> --- | 1-3,7 | RECHERCHIERTE SACHGEBIETE (Int.Cl.6) <br><br> C07D |
| A | PATENT ABSTRACTS OF JAPAN <br> vol. 2 no. 93 (C-78) ,29.Juli 1978 <br> & JP-A-53 053686 (UNITIKA KK) <br> * Zusammenfassung * <br> --- | 1-3,7 | |
| A | EP-A-0 522 235 (BASF CORP.) <br> * Beispiele * <br> ----- | 1 | |

Der vorliegende Recherchenbericht wurde für alle Patentansprüche erstellt

| Recherchenort | Abschlußdatum der Recherche | Prüfer |
|---|---|---|
| BERLIN | 16.Mai 1995 | Van Amsterdam, L |

EPO FORM 1503 03.82 (P04C03)